# EUROPÄISCHE PATENTSCHRIFT

(11) **EP 2 736 431 B1**
(45) Veröffentlichungstag und Bekanntmachung des Hinweises auf die Patenterteilung: **06.09.2017**
(21) Anmeldenummer: 11787580.7
(22) Anmeldetag: 27.07.2011
(51) Int. Cl.: A61B 17/80, A61B 17/00, A61L 27/58, A61F 2/02, A61F 2/28, B22F 1/00, A61L 27/04, A61L 31/14, A61F 2/30, B22F 9/06, C22C 49/14

(54) **IMPLANTAT**
IMPLANT
IMPLANT

(43) Veröffentlichungstag der Anmeldung: 04.06.2014
(73) Patentinhaber: Fraunhofer Gesellschaft zur Förderung der angewandten Forschung E.V., 80686 München (DE); Medizinische Hochschule Hannover (MHH), 30625 Hannover (DE)
(72) Erfinder: ANDERSEN, Olaf, 01097 Dresden (DE); MORGENTHAL, Ingrid, 01187 Dresden (DE); STUDNITZKY, Thomas, 01309 Dresden (DE); WITTE, Frank, 30171 Hannover (DE)
(74) Vertreter: Pfenning, Meinig & Partner mbB
(86) Internationale Anmeldenummer: PCT/DE2011/001539
(87) Internationale Veröffentlichungsnummer: WO 2013/013648

(56) Entgegenhaltungen:
- WO-A2-03/055537
- DE-A1- 10 241 572
- DE-A1- 19 712 625
- DE-B3-102008 046 197
- Olaf Andersen: "Aluminiumfaser-Strukturen für Adsorptions-Kältemaschinen", , 6. April 2011 (2011-04-06), Seiten 1-2, XP55024408, Gefunden im Internet: URL:http://www.ifam-dd.fraunhofer.de/conte nt/dam/ifam-dd/de/documents/Dokumente ZMW/Fachinfo_Adsorptionskaelte.pdf [gefunden am 2012-04-13]
- GÜNTER STEPHANI ET AL: "New multifunctional lightweight materials based on cellular metals - manufacturing, properties and applications", JOURNAL OF PHYSICS: CONFERENCE SERIES, Bd. 165, 1. Mai 2009 (2009-05-01), Seite 012061, XP55024406, DOI: 10.1088/1742-6596/165/1/012061

## Beschreibung

Die Erfindung betrifft Implantate, die als temporäre Implantate in Lebewesen implantiert werden und sich durch Bioresorption über einen Zeitraum im Körper zersetzen.

Prinzipiell sind solche Implantate aus den unterschiedlichsten Werkstoffen bekannt. In der DE 10 2008 037 204 A1 ist eine Platte zur Fixierung von Knochenfragmenten beschrieben, die aus einem gepressten Formkörper aus Draht besteht. Vorzugsweise soll dies ein gepresstes Gestrick sein. Unter anderem sind auch Magnesium oder eine Magnesiumlegierung als geeignete Werkstoffe für die eingesetzten Drähte darin genannt.

Bei der Herstellung sollen die Drähte als Maschenware gefaltet und/oder aufgerollt und dann durch Pressen zu einem Formkörper verarbeitet werden.

Dabei ist aber die Möglichkeit der geometrischen Gestaltung eines solchen Implantats begrenzt und es sind jeweils ein bestimmtes Form- und Presswerkzeug für ein Implantat erforderlich. Es ist auch beschrieben, wie Bohrungen hergestellt werden können. Hierfür ist in der Regel ein bestimmtes Einlegen der Drähte in das Formwerkzeug erforderlich, bei dem die Anordnung einer Bohrung berücksichtigt wird. Außerdem ist ein entsprechendes, der gewünschten Form des Implantates angepasstes Presswerkzeug erforderlich. Die eingesetzten Drähte müssen eine bestimmte ausreichende Länge haben, um eine sichere Verbindung zu erreichen, was bei Magnesium und Magnesiumlegierungen, insbesondere bei der gewünschten Verformung beim Pressen problematisch ist.

Eine nachträgliche Formgebung oder gar die Herstellung mehrerer Implantate aus einem Rohling ist nicht mehr möglich.

Für bestimmte Anwendungsfälle wirkt sich auch die flexible Verformbarkeit solcher Implantate durch deren Nachgiebigkeit nachteilig aus, da die Drähte nicht fest miteinander verbunden sind.

So ist aus DE 102 41 572 A1 ein Stütz- oder Halteteil zum Einbringen in ein Knochenteil bekannt, das aus Magnesium als abbaubares Material bestehen soll. Darin sollen Hohlräume vorhanden sein, die zur Aufnahme eines Medikamentes, insbesondere eines das Knochenwachstum anregenden Stoffes vorgesehen sind.

Von Olaf Andersen sind in "Aluminiumfaser-Strukturen für Adsorptions-Kältemaschinen"; 06. April 2011 (2011-04-06), Seiten 1 und 2, XP55024408 sowie in DE 197 12 625 A1 aus Metallfasern gebildete Strukturele mente bekannt.

DE 10 2008 046 197 B3 betrifft ein degradierbares Implantat, ein Verfahren zu dessen Herstellung und seine Verwendung. Die Degradierbarkeit und die Gewebeverträglichkeit sollen durch eine poröse Beschichtung verbessert werden.

Aus WO 03/055537 A2 sind medizinische Implantate, Prothesen, Prothesenteile, medizinische Instrumente, Geräte und Hilfsmittel aus einem mit Halogenid modifizierten Werkstoff bekannt.

Es ist daher Aufgabe der Erfindung, Möglichkeiten anzugeben, mit denen bioresorbierbare Implantate, die flexibel und kostengünstig herstellbar sind und bei denen keine physiologischen Bedenken bei einer Implantation auftreten, zur Verfügung gestellt werden können.

Erfindungsgemäß wird diese Aufgabe mit einem Implantat, das die Merkmale des Anspruchs 1 aufweist, gelöst. Es kann mit einem Verfahren nach Anspruch 10 hergestellt werden. Vorteilhafte Ausgestaltungen und Weiterbildungen der Erfindung sind mit in untergeordneten Ansprüchen bezeichneten Merkmalen realisierbar.

Das erfindungsgemäße Implantat besteht aus Magnesium oder einer Magensium-Basislegierung. Es ist aus Fasern aus Magnesium oder aus einer Magnesium-Basislegierung gebildet, die durch lokal zueinander beabstandete Sinterbrücken miteinander so verbunden sind, dass sie einen offenporigen Körper bilden.

Die Fasern sollten eine Länge im Bereich 2 mm bis 15 mm, bevorzugt im Bereich 5 mm bis 10 mm und/oder einen Außendurchmesser im Bereich 0,05 mm bis 0,5 mm aufweisen. Dadurch können sie kostengünstig hergestellt und einfach weiter verarbeitet werden.

Vorteilhaft ist es, wenn die Magnesium-Basislegierung mit mindestens einem Metall gebildet ist, mit dem eine flüssige Phase bei einer Temperatur unterhalb der Schmelztemperatur des reinen Magnesiums ausgebildet werden kann. Auch dadurch besteht die Möglichkeit, dass der Anteil der gebildeten flüssigen Phase kleiner 20 % gehalten werden kann, Wodurch einmal eine sichere Verbindung der Fasern über die Sinterbrücken an den Berührungspositionen und auch eine ausreichende Festigkeit des Faserverbundes erreichbar ist.

So können in einer Magnesium-Basislegierung neben Magnesium mindestens ein Metall enthalten sein, das ausgewählt ist aus Y, Zn, Ca, Mn, Pd, Ag, Sr, Bi, Si, Pr, Ga, Sc, Zr, Ce, Eu, La, Nd, Na und Li.

Dabei soll unter einer Magnesium-Basislegierung eine Legierung verstanden werden, in der der Anteil an Magnesium größer als 50 %, bevorzugt größer 75 % ist. In der Legierung sollten kein Aluminium, kein Kupfer und auch kein Nickel, bis auf Spuren, die maximal 1 %, bevorzugt maximal 0,1 % ausmachen, enthalten sein.

Das Implantat sollte eine Porosität im Bereich 50 % bis 95 % aufweisen. Allein oder zusätzlich dazu sollte es eine volumenspezifische Oberfläche im Bereich 5000 m²/m³ bis 50000 m²/m³ aufweisen.

Vorteilhaft ist es, wenn an der Oberfläche der Fasern zumindest bereichsweise eine Magnesiumoxidschicht und/oder eine Fluoridschicht ausgebildet ist. Diese sollte vorzugsweise eine Schichtdicke von mindestens 0,1 µm aufweisen. Mit einer solchen Oxid- oder Fluoridschicht kann die Degradationsgeschwindigkeit beeinflusst werden, mit der das Implantat in einem lebenden Körper resorbiert wird, so dass es nach einem entsprechend vorgebbaren Zeitraum vollständig und somit rückstandslos resorbiert und dadurch keine operative Entfernung mehr erforderlich ist. Je dicker diese Oxid- oder Fluoridschicht ist je länger kann die vollständige Resorption verzögert werden. Es können auch Bereiche an einem Implantat vorhanden sein an denen eine Magnesiumoxidschicht ausgebildet ist und andere Oberflächenbereiche mit einer Magnesiumfluoridschicht versehen sind. Auch dadurch kann eine unterschiedliche Zeit für eine vollständige Degradation solcher Bereiche eines Implantats eingehalten werden. Hierfür kann ein bereichsweise an der Oberfläche mit einer Magnesiumoxidschicht versehenes Implantat teilweise in eine Fluoridlösung eingetaucht und dann getrocknet werden, so dass dadurch der eingetauchte Bereich zusätzlich mit einer Fluoridschicht versehen wird.

Geeignete Fluoride sind beispielsweise Magnesium- und Natriumfluorid.

Bei einer bereichsweisen Beschichtung werden unbeschichtete oder Bereiche mit kleinerer Oxidschichtdicke schneller resorbiert, als beschichtete Bereiche, so dass auch eine lokal gezielte Beeinflussung möglich ist.

An einem Implantat können auch Bereiche mit unterschiedlicher Porosität und Festigkeit vorhanden sein, so dass eine Anpassung an eine jeweils gewünschte Applikation möglich ist. Dabei kann die Packungsdichte von Fasern und somit die Porosität in den jeweiligen Bereichen variieren. Bei erhöhter Packungsdichte, also mehr Fasern pro Volumeneinheit kann die Festigkeit in einem solchen Bereich erhöht und in anderen Bereichen entsprechend reduziert werden. Auch das Einwachsverhalten von Gewebe kann so lokal gezielt beeinflusst werden.

Bei der Herstellung eines Implantats aus Magnesium oder einer Magnesium-Basislegierung kann so vorgegangen werden, dass Fasern durch ein Schmelzextraktionsverfahren aus einer Magnesiumschmelze oder einer Magnesium-Basislegierungsschmelze in einer inerten Atmosphäre hergestellt werden. Prinzipiell ist das Verfahren der Schmelzextraktion u.a. in der DE 100 00 097 A1 und der DE 10 2006 005 510 A1 neben verschiedenen möglichen Ausführungsformen mit beschrieben.

Die so hergestellten Fasern werden in einer Schüttung auf eine Unterlage oder in eine Form, die aus einem inerten Werkstoff besteht, eingelegt und anschließend erfolgt dann in einer inerten Atmosphäre eine Sinterung, bei der die Fasern über lokal voneinander beabstandete Sinterbrücken miteinander verbunden werden.

Bei der Herstellung mit einer Magnesium-Basislegierung kann die Sinterung unterhalb der Schmelztemperatur des reinen Magnesiums durchgeführt werden, da die gewünschte flüssige Schmelzphase, die für ein wünschenswertes Flüssigphasensintern im Bereich der auszubildenden Sinterbrücken, bei einer geeignet ausgewählten Magnesium-Basislegierung, bei einer entsprechend erniedrigten Temperatur erreicht werden kann.

Die bereits erwähnte Magnesiumoxidschicht an der Oberfläche der Fasern kann mittels einer anodischen Oxidation, einer plasmachemischen Oxidation oder einer thermischen Oxidation ausgebildet werden. Dies erfolgt im Anschluss an den Sinterprozess.

Mit der Erfindung ist es möglich, relativ großformatige offenporige Körper mit den verbundenen Fasern herzustellen, die dabei auch Halbzeuge darstellen und durch geeignete Schneid- oder Trennverfahren in mehrere einzelne Implantate zerteilt werden können, was den Herstellungsaufwand reduziert. Durch solche Verfahren kann aber auch die äußere Geometrie und/oder Dimensionierung eines Implantats an die jeweilige Anforderung angepasst werden. Als ein geeignetes Trennverfahren hat sich der Einsatz von Laserstrahlung herausgestellt, wobei auch in diesem Fall eine Schutzgasatmosphäre (z.B. Ar) eingehalten werden sollte. Auch nach dem Abtrennen von Teilen oder Bereichen des gesinterten offenporigen Körpers bleibt dessen Festigkeit zumindest im Wesentlichen erhalten. Dies betrifft sowohl die Druck-, wie auch die Zugfestigkeit. Außerdem bleibt der Verbund der miteinander versinterten Fasern erhalten. Diese Formgebung sollte jedoch vor einer ggf. gewünschten Ausbildung einer Magnesiumoxidschicht erfolgen.

Neben der bereits erwähnten Verunreinigung mit weiteren Metallen, wie dies z.B. Aluminium ist, kann auch auf andere Fremd- oder Hilfsstoffe (Tracer) verzichtet werden. Das Implantat besteht zumindest nahezu ausschließlich aus Magnesium, der jeweiligen Magnesium-Basislegierung und ggf. der Magnesiumoxidschicht und/oder Fluoridschicht. Dadurch kann auf aufwändige Zulassungsverfahren verzichtet werden, da hierfür die entsprechenden Biokompatibilitätsnachweise bereits erbracht worden sind und eine Produktzulassung einfach und mit geringem Aufwand erlangt werden kann. Insbesondere sind keine organischen Komponenten oder Platzhalter vorhanden, da diese im Gegensatz zur Herstellung von Metallschaum, nicht bei der Herstellung erforderlich sind. Es ist kein Nachweis erforderlich, dass organische oder andere Rückstände nicht im fertig hergestellten Implantat enthalten sind.

Im Gegensatz zu Schäumen ist eine vollständige offene Porosität möglich.

Ein bei der Erfindung einsetzbarer offenporiger Körper, als erfindungsgemäßes Implantat, kann auch auf ein weiteres, anderes bevorzugt metallisches Implantat (beispielsweise eine Prothese), das nicht oder nur geringfügig porös ist, aufgebracht und bevorzugt stoffschlüssig mit diesem verbunden werden. Dadurch kann das Einwachsen von Gewebe oder Knochen sowie eine knöcherne Verankerung verbessert werden. Ein erfindungsgemäßes Implantat kann dabei als temporäres Implantat bezeichnet werden, dass dann mit einem permanenten Implantat (z.B. einem Hüftprothesenschaft) verbunden wird. Das in Folge der Degradation temporäre Mg-Fasergeflecht ermöglicht ein besseres Einwachsen eines Knochens und es kann dadurch eine schnellere sichere Verankerung des Hüftschaftes erreicht werden. Nach dem Auflösen des erfindungsgemäßen Implantats steht die Grundform (z.B. die raue Oberfläche des Hüftschaftes) mit dem Knochen endgültig in Kontakt.

Nachfolgend soll die Erfindung an Hand von Beispielen näher erläutert werden.

### Beispiel 1:

Aus einer Legierung MgY4 (4 Masse-% Yttrium Rest Magnesium) werden mittels des Schmelzextraktionsverfahrens metallische Kurzfasern hergestellt. Die Magnesium-Basislegierung wird in einem Tiegel aus Ni-freiem Stahl induktiv bei einer Temperatur von 680 °C erschmolzen. Die Extraktionswalze taucht in das Schmelzbad ein, die Schmelze benetzt die Walze und durch die rotierende Walze werden kleine Mengen Schmelze mitgerissen, erstarren an der gekühlten Walze, lösen sich von der Walzenoberfläche ab und werden in einem Behälter aufgefangen. Weitere Parameter, wie Tiegelhub und Rotationsgeschwindigkeit bestimmen neben dem Kerbabstand der Walze die Geometrie der hergestellten Kurzfasern, wie Faserlänge und mittleren Faserquerschnitt/Außendurchmesser/Flächendiagonale. Aufgrund der Erstarrung an der Kante der Walzenoberfläche haben die Fasern einen typischen Halbmond- oder Sichelquerschnitt und die Schnellerstarrung führt zu dem charakteristischen Erstarrungsgefüge.

Zur Vermeidung der Oxidbildung auf der Faseroberfläche wird die Schmelzextraktionsanlage mit Argon-Schutzgas geflutet, so dass die Fasern in einer inerten Atmosphäre extrahiert werden.

Problematisch ist das Abdampfen von Mg aufgrund des dem Werkstoff eigenen hohen Dampfdruckes, wodurch die Kontrolle der Extraktionsparameter über das Sichtfenster erschwert wird. Mit einer Variante 2 (Beispiel 2), dem Abdecken der Schmelze mit lose aufliegenden Eisenbasis-Hohlkugeln gelang es deutlich, das Abdampfen im Bereich der Extraktionsanlage zu reduzieren.

Tabelle 1 enthält die wichtigsten Daten der Schmelzextraktion verschiedener MgY4-Faserchargen und Tabelle 2 die charakteristischen Faserkennwerte.

Im Beispiel 1 werden MgY4-Fasern ohne Schmelzbadabdeckung hergestellt, die einen mittleren kreisäquivalenten Durchmesser von 216 ± 68 µm und eine mittlere Faserlänge von 7,2 mm aufweisen. Diese Fasern zeichnen sich durch eine hohe Reinheit vergleichbar dem des Ausgangsmaterials (Schmelzingot) aus. In der Gesamtanalyse wird Sauerstoff mit ≤ 0,002 % bestimmt.

Um einen hochporösen, offenzelligen Sinterkörper aus Mg-Kurzfasern zu erhalten wird vor dem eigentlichen Sinterprozess eine definierte Faserschüttung hergestellt. Dies erfolgt mittels eines Siebes (Siebtrommel für große Proben). Die Fasern werden vereinzelt und gleichmäßig Schicht für Schicht auf ein Sintersubstrat gebracht. Anschließend werden die Fasern unter Anwendung von Parametern, die es ermöglichen einen hochporösen und zugleich festen Sinterverbund zu erzielen, im Ofen gesintert. Die Sinterung erfolgt unterhalb der Schmelztemperatur der Magensium-Basislegierung bei einer Temperatur von 625 °C bis 630 °C, bei der sich temporäre flüssige Phasen bilden. Ein Flüssigphasenanteil zwischen 10 % und 20 % im Bereich der Sintertemperatur wirkt sich günstig auf das Erzielen eines stabilen Sinterverbundes aus und ist Voraussetzung für das Sintern von Werkstoffen mit Oberflächenoxidbildung, wie dies bei Mg der Fall ist.

Die Fasern sollten nicht mit dem Sintersubstrat reagieren, was gleichzeitig bedeutet, dass sich die gesinterte Probe leicht von der Sinterunterlage lösen lassen soll. Aus diesem Grund wurden die MgY4-Fasern auf eine inerte Unterlage aus Tantalblech in der Weise deponiert, dass die Struktur nach dem Sintern die gewünschte Porosität von 75 % aufweist. Unterlage und Deckblech aus Tantal sichern, dass sich die chemische Zusammensetzung der Fasern während der Sinterung nicht verändert.

Es wurde eine Schüttung in den erforderlichen Raumausdehnungen hergestellt, so dass nach dem Sintern die Abmessung 100 mm * 50 mm * 3 mm in der gewünschten Porosität erreicht werden kann.

Die Sinterung erfolgte im Schutzgasofen (Quarzglasrohr) unter hochreinem Argon 6.0 durch Aufheizen der Probe mit 10 K/ min bis auf 600 °C, bei einer Haltezeit von 1 min und anschließendem weiterem Aufheizen mit 3 K/ min bis zur Sintertemperatur Tₘₐₓ von 628 °C. Um einen Sauerstoffeintrag aus der Ofenatmosphäre in die Fasern zu vermeiden, wurde Titan als

### Gettermaterial eingesetzt.

Die gesinterte Struktur zeichnet sich durch stabile Sinterkontakte im Bereich der Sinterbrücken, eine hohe mittlere Porosität von 73,5 % und sehr gute Bearbeitbarkeit aus. Die Bearbeitung zu definierten offenporigen Formkörpern kann durch Sägen (trocken) oder Laserschneiden erfolgen. Offenporige Körper definierter Geometrie, z. B. Ronden mit Durchmesser 10 mm, wurden mittels Laserschneiden unter Argon-Schutzgas hergestellt.

Zur Beurteilung der Degradation wurden Korrosionsuntersuchungen unter Verwendung von Dulbecco's Modifiziertem Eagle Medium (DMEM), Lieferant Fa. Biochrom GmbH Berlin, über einen Zeitraum von bis zu 7 Tagen und einer Temperierung bei 37 °C durchgeführt. Als Maß für die Korrosion wurde die Wasserstoffentwicklung (Volumen V in ml) gemessen. Vergleichsuntersuchungen an kompaktem Material aus der Legierung MgY4 zeigten, dass die Degradation der offenporigen Körper absolut gesehen, zu einer stärkeren Wasserstofffreisetzung führt. Berücksichtigt man jedoch die große Oberfläche der porösen Körper und bezieht das Wasserstoffvolumen auf die Probenoberfläche (V_{N} in ml/ cm²), so schneiden diese besser als der kompakte Werkstoff ab.

### Beispiel 2:

Ein nach Beispiel 1 gesinterter offenporiger Körper wurde nach erfolgter Sinterung durch Sauerstoffeinwirkung bei Temperatur unterhalb der Sintertemperatur oberflächlich oxidiert. Da die Oxidation nach dem Sintern erfolgt, schädigt die ausgebildete Oxidschicht nicht die Sinterbrücken zwischen den Fasern. Die Korrosionsprüfung in DMEM zeigt für diese Probe eine verbesserte Beständigkeit, d. h. verlangsamte Auflösung (Degradation) des Faser-Verbundes.

### Beispiel 3:

Analog Beispiel 1 werden MgY4-Fasern hergestellt. Es wurde bei der Schmelzextraktion aber eine Schmelzbadabdeckung, indem Eisenbasis-Hohlkugeln auf das zu schmelzende Metall lose, jedoch die Oberfläche bedeckend, aufgelegt werden, eingesetzt. Nach dem induktiven Erschmelzen der MgY-Legierung schwimmen die Hohlkugeln auf dem Schmelzbad, ohne sich in diesem zu lösen. Sie reduzieren damit deutlich das Abdampfen der Schmelze innerhalb der Extraktionsanlage.

Die auf diese weise hergestellten Fasern aus MgY4 weisen einen mittleren kreisäquivalenten Durchmesser von 187 ± 63 µm und eine mittlere Faserlänge von 5,8 mm auf. Diese Fasern zeichnen sich ebenfalls durch eine hohe Reinheit aus. Die chemische Analyse der Fasern im Vergleich zum Schmelzingot MgY4 zeigt keine signifikanten Änderungen des Gehaltes an Y und keine Erhöhung der nicht erwünschten Elemente Al, Cu oder Fe. Letztere sind im Ausgangmaterial in Gehalten < 0,004 bzw. 0,005 % vorhanden.

### Beispiel 4:

Analog zu Beispiel 1 werden Fasern der Magnesium-Basislegierung MgY4 zu einer hochporösen Struktur geschüttet und gesintert. In Abwandlung zu Beispiel 1 wurden die Fasern auf einer Unterlage aus Tantalblech, das mit einer MgO-Suspension beschichtet wurde, gesintert. Vor der ersten Verwendung wird die Suspension, die aus Pulver Magnesia 298, dispergiert in Ethanol, besteht, bei 800 °C unter Argon-Schutzgas ausgebrannt. Damit wird eine hochreine Beschichtung erzielt. Vorteilhaft an dieser MgO-Beschichtung ist, dass sich der mit den versinterten Fasern gebildete offenporige Körper nach dem Sintern leicht lösen lässt. Das nachfolgende Handling wird deutlich erleichtert. Eine infolge des Sinterprozesses eingetretene mögliche "Verankerung" der feinen MgO-Pulverteilchen an der äußeren Faserzone des offenporigen Körpers wirkt sich vorteilhaft auf das Korrosionsverhalten aus. Es wurde eine leichte Verringerung der Korrosionsrate festgestellt, so dass die Degradation über einen größeren Zeitraum erfolgen sollte.

### Beispiel 5:

Analog Beispiel 1 werden Fasern der Magnesium-BasisLegierung MgY4 zu einer hochporösen Struktur geschüttet und gesintert. Der gesinterte offenporige Körper wird einer plasmachemischen Oxidation unterworfen. Es bildet sich eine Oxidschicht auf der Oberfläche der Faserstruktur aus. Diese bewirkt, dass die Korrosion in DMEM (Ausführung s. Beispiel 1) deutlich langsamer verläuft.

**Tabelle 1: Extraktionsbedingungen; Fasern MgY4 (3 Beispiele)**

| **Fasercharge** | **Temperatur** | **Tiegelhub** | **Walzen-Geschwind.** | **Versuchszeit** | **Schmelzbadabdeckung** |
|---|---|---|---|---|---|
| | **[°C]** | **[µm/s]** | **[m/s]** | **[min]** | |
| (1) D114 | 680 | 20,0 | 3,0 | 10 | Ohne |
| (2) V621 | 800 | 20,0 | 5 | 30 | Hohlkugeln |
| (3) V622 | 800 | 20,0 | 4,2 | 30 | Hohlkugeln |

**Tabelle 2: Faserkennwerte aus Bildanalyse (*) und Scanneranalyse (**)**

| **Fasercharge** | **Material** | **Dichte** | **mittlere Dicke** | **mittlere Länge** | **massenspezifische Oberfläche** | **äquiv. Kreis-Durchmesser** |
|---|---|---|---|---|---|---|
| | | **[g/cm³]** | **[µm]** | **[mm]** | **Aₘ [m²/g]** | **[µm]** |
| | | | (**) | (**) | (*) | (*) |
| (1) D 114 | MgY | 1,78 | 224 | 7,2 ± 1,5 | 0,01175 | 216 ± 68 |
| (2) V621 | MgY4 | 1,78 | 243 | 5,8 ± 1,4 | 0,01349 | 187 ± 63 |
| (3) V622 | MgY4 | 1,78 | 179 | 5,4 ± 1,7 | 0,01334 | 166 ± 47 |

### Beispiel 6:

Die Basislegierung MgY4 (4 Masse-% Yttrium) wurde unter Zusatz von hochreinen Ca-Granulat in einer Hochvakuum-Schmelz- und Gießanlage zu einer MgYCa-Legierung erschmolzen. Für eine gute Durchmischung der Komponenten wurden die Bestandteile mehrmals umgeschmolzen. Der fertige Schmelzingot wurde analysiert bezüglich der Elementgehalte und eventuell enthaltener Verunreinigungen. Die Analysen ergaben im Mittel 2,6 % Yttrium und 1,6 % Kalzium. Für die nicht erwünschten Begleitelemente Cu und Fe wurden Gehalte < 0,004 bzw. < 0,003 % ermittelt. Der Sauerstoffgehalt betrug max. 0,01 %.

Analog zum Beispiel 3 werden Fasern aus dieser Legierung MgY2,6Ca1,6 hergestellt. D. h. die Faserherstellung erfolgte mit Schmelzbadabdeckung, indem Eisenbasis-Hohlkugeln auf dem Schmelzbad und während der Schmelzextraktion aufliegen.

Die auf diese Weise hergestellten Fasern aus MgY2,6Ca1,6 (Charge V620) weisen einen mittleren kreisäquivalenten Durchmesser von 166 ± 75 µm und eine mittlere Faserlänge von 7,4 mm auf. Diese Fasern zeichnen sich ebenfalls durch eine hohe Reinheit aus. Die chemische Analyse der Fasern im Vergleich zum Schmelzingot MgY4 zeigt keine signifikanten Änderungen des Gehaltes an Y und keine nennenswerte Erhöhung der nicht erwünschten Elemente Cu oder Fe. Für Cu wurden 0,004 % und für Fe 0,007 % ermittelt.

Die Daten zu den Fasern können den Tabellen 3 und Tabelle 4 entnommen werden.

**Tabelle 3: Extraktionsbedingungen Fasern MgY2,6Ca1,6**

| **Fasercharge** | **Temperatur** | **Tiegelhub** | **Walzen-Geschwind.** | **Versuchszeit** | **Schmelzbadabdeckung** |
|---|---|---|---|---|---|
| | **[°C]** | **[µm/s]** | **[m/s]** | **[min]** | |
| V620 | 800 | 20,0 | 3,5 | 30 | Hohlkugeln |

**Tabelle 4: Faserkennwerte aus einer Bildanalyse (*) und Scanneranalyse (**)**

| **Fasercharge** | **Material** | **Dichte** | **mittlere Dicke** | **mittlere Länge** | **massenspezifische Oberfläche** | **äquiv. Kreis-Durchmesser** |
|---|---|---|---|---|---|---|
| | | **[g/cm³]** | **[µm]** | **[mm]** | **Aₘ [m²/g]** | **[µm]** |
| | | | (**) | (**) | (*) | (*) |
| V620 | MgY2,6Ca1,6 | 1,74 | 296 | 7,4 ± 1,3 | 0,01531 | 166 ± 75 |

### Beispiel 7:

Analog zu Beispiel 6 hergestellte Fasern werden analog Beispiel 1 zu einer hochporösen Struktur geschüttet und gesintert. Unter Beachtung des ternären Phasendiagramms und thermodynamischer Berechnungen zur Abschätzung der Sintertemperaturen mittels der Software PANDAT liegt für die Legierung MgY2,6Ca1,6 die Sintertemperatur im Bereich von 550 °C bis 590 °C, was einem Flüssigphasenanteil von 10 bis 20 % entspricht.

Eine Sinterung der Fasern aus MgY2,6Ca1,6, geschüttet auf eine inerte Unterlage aus Tantalblech, wurde bei 580 °C durchgeführt. Die Sinterung erfolgte wie im Beispiel 1 beschrieben im Schutzgasofen (Quarzglasrohr) unter hochreinem Argon 6.0 durch Aufheizen der Probe mit 10 K/ min bis 550 °C, Haltezeit 1 min, und anschließendes Aufheizen mit 3 K/ min bis zur Sintertemperatur Tₘₐₓ von 580 °C, Haltezeit 20 min.

Die gesinterte Struktur zeichnet sich durch stabile Sinterkontakte, eine hohe Porosität von 61 % im Probenmittel und sehr gute Bearbeitbarkeit aus. Die Bearbeitung zu definierten Formkörpern in Form von Ronden Durchmesser 10 mm erfolgte durch Laserschneiden. Die gesinterte Struktur zeichnet sich durch eine hohe Reinheit aus (Sauerstoffgehalt max. 0,001 %). Aufgrund der geänderten Zusammensetzung (2,6 % Y; 1,6 % Ca) ist die im Korrosionsversuch messbare Degradationsgeschwindigkeit etwas höher als bei dem nach Beispiel 1 hegestellten Implantat.

Die in den Beispielen 1 bis 7 hergestellten Implantate können wie folgt gereinigt und dann auch beschichtet werden. Für die Reinigung werden 20ml Glycerol (85%), 5ml Salpetersäure (65%), 5ml Essigsäure (100%) vermischt und das gesinterte Implantat 30 sec in die Lösung getaucht. Anschließend wird es zweimal jeweils 60 sec in 100%-igem Ethanol im Ultraschallbad gereinigt und danach mit einem Druckgasreiniger angeströmt. Im Anschluss daran erfolgt ein vollständiges Trocknen im Vakuumschrank bei einer Temperatur von 50°C über einen Zeitraum von ca. 10 min.

Das so gereinigte und getrocknete Implantat kann in eine gesättigte Natriumfluorid-Lösung eingetaucht werden. Der Lösung werden 4 g in 100ml Aqua ad iniectabilia (Fa Braun, 100ml Glasflasche) zugegeben und die Mischung wird in einem Glasgefäß angesetzt und darin über einen Zeitraum von 1 bis 2 h gerührt.

Der pH-Wert wird durch Zugabe von NaOH auf 11,5 eingestellt. In die so erhaltene Lösung kann das Implantat eingelegt und eine Natriumfluorid-Beschichtung an der Oberfläche ausgebildet werden. Nach einer Trocknung in einem Vakuumschrank hatte die NatriumFluorid-Beschichtung eine mittlere Schichtdicke von mindestens 0,1 µm. Es konnte mit der Beschichtung eine deutliche Verringerung der Korrosionsrate im Vergleich zu einer unbeschichteten Faserstruktur erreicht werden.

## Patentansprüche

1. Implantat, das aus Magnesium oder einer Magnesium-Basislegierung besteht, **dadurch gekennzeichnet, dass** es aus Fasern aus Magnesium oder aus einer Magnesium-Basislegierung besteht, die durch lokal zueinander beabstandete Sinterbrücken miteinander verbunden sind und einen offenporigen Körper bilden.

2. Implantat nach Anspruch 1, **dadurch gekennzeichnet, dass** die Fasern eine Länge im Bereich 2 mm bis 15 mm, bevorzugt im Bereich 5 mm bis 10 mm und/oder einen Außendurchmesser im Bereich 0,05 mm bis 0,5 mm aufweisen.

3. Implantat nach Anspruch 1 oder 2, **dadurch gekennzeichnet, dass** die Magnesium-Basislegierung mit mindestens einem Metall gebildet ist, mit dem eine flüssige Phase bei einer Temperatur unterhalb der Schmelztemperatur des reinen Magnesiums ausbildbar ist.

4. Implantat nach einem der vorhergehenden Ansprüche, **dadurch gekennzeichnet, dass** in der Magnesium-Basislegierung mindestens ein Metall enthalten ist, das ausgewählt ist aus Y, Zn, Ca, Mn, Pd, Ag, Sr, Bi, Si, Pr, Ga, Sc, Zr, Ce, Eu, La, Nd, Na und Li.

5. Implantat nach einem der vorhergehenden Ansprüche, **dadurch gekennzeichnet, dass** es eine Porosität im Bereich 50 % bis 95 % und/oder eine volumenspezifische Oberfläche im Bereich 5000 m²/m³ bis 50000 m²/m³ aufweist.

6. Implantat nach einem der vorhergehenden Ansprüche, **dadurch gekennzeichnet, dass** an der Oberfläche der Fasern zumindest bereichsweise eine Magnesiumoxidschicht ausgebildet ist, die vorzugsweise eine Schichtdicke von mindestens 0,1 µm aufweist.

7. Implantat nach einem der vorhergehenden Ansprüche, **dadurch gekennzeichnet, dass** an der Oberfläche der Fasern zumindest bereichsweise eine Fluoridschicht ausgebildet ist, die vorzugsweise eine Schichtdicke von mindestens 0,1 µm aufweist

8. Implantat nach einem der vorhergehenden Ansprüche, **dadurch gekennzeichnet, dass** Bereiche mit unterschiedlicher Porosität und Festigkeit vorhanden sind.

9. Implantat nach einem der vorhergehenden Ansprüche, **dadurch gekennzeichnet, dass** es mit einem weiteren Implantat verbunden ist.

10. Verfahren zur Herstellung eines Implantats aus Magnesium oder einer Magnesium-Basislegierung, bei dem
Fasern durch ein Schmelzextraktionsverfahren aus einer Magnesiumschmelze oder einer Magnesium-Basislegierungsschmelze in einer inerten Atmosphäre hergestellt werden;
die Fasern in einer Schüttung auf eine Unterlage oder in eine Form, die aus einem inerten Werkstoff besteht, eingelegt und anschließend in einer inerten Atmosphäre eine Sinterung erfolgt, bei der die Fasern über lokal voneinander beabstandete Sinterbrücken miteinander verbunden werden.

11. Verfahren nach Anspruch 10, **dadurch gekennzeichnet, dass** bei der Herstellung mit einer Magnesium-Basislegierung, die Sinterung unterhalb der Schmelztemperatur des reinen Magnesiums durchgeführt wird, wobei der Anteil der gebildeten flüssigen Phase bevorzugt kleiner 20 % gehalten werden soll.

12. Verfahren nach Anspruch 10 oder 11, **dadurch gekennzeichnet, dass** mittels einer anodischen Oxidation, einer plasmachemischen Oxidation oder einer thermischen Oxidation an der Oberfläche der Fasern zumindest bereichsweise eine Magnesiumoxidschicht ausgebildet wird.

13. Verfahren nach einem der Ansprüche 10 bis 12, **dadurch gekennzeichnet, dass** das Implantat mittels eines Trenn- oder Schneidverfahrens, insbesondere durch Laserschneiden in eine Endform gebracht wird.

## Claims

1. Implant consisting of magnesium or a magnesium master alloy, **characterized in that** it consists of fibers of magnesium or a magnesium master alloy that are connected to each other by sintered bridges that are locally spaced vis-a-vis one another and form an open-pored body.

2. Implant according to Claim 1, **characterized in that** the fibers have a length ranging from 2 mm to 15 mm, preferably ranging from 5mm to 10 mm and/or an outer diameter ranging from 0.05 mm to 0.5 mm.

3. Implant according to Claim 1 or 2, **characterized in that** the magnesium master alloy is formed with at least one metal with which a liquid phase can be achieved at a temperature lower than the melting temperature of pure magnesium.

4. Implant according to one of the preceding Claims, **characterized in that** the magnesium master alloy contains at least one metal chosen from Y, Zn, Ca, Mn, Pd, Ag, Sr, Bi, Si, Pr, Ga, Sc, Zr, Ce, Eu, La, Nd, Na and Li.

5. Implant according to one of the preceding Claims, **characterized in that** it has a porosity ranging from 50 % to 95 % and/or a volume-specific surface ranging from 5000 m²/m³ to 50000 m²/m³.

6. Implant according to one of the preceding Claims, **characterized in that** at the surface of the fibers at least in certain areas a magnesium oxide layer is formed having preferably a layer thickness of at least 0.1 µm.

7. Implant according to one of the preceding Claims, **characterized in that** at the surface of the fibers at least in certain areas a fluoride layer is formed having preferably a layer thickness of at least 0.1 µm.

8. Implant according to one of the preceding Claims, **characterized in that** areas with different porosity and strength are present.

9. Implant according to one of the preceding Claims, **characterized in that** it is connected to another implant.

10. Process for manufacturing an implant from magnesium or a magnesium master alloy in which fibers are produced with the help of a melt extraction process from molten magnesium or a molten magnesium master alloy in an inert atmosphere;
the fibers are deposited in bulked form onto a substrate or into a mold comprised of an inert material, and subsequently a sintering process takes place in an inert atmosphere during which the fibers are connected with each other through sintering bridges that are locally spaced vis-a-vis one another.

11. Process according to Claim 10, **characterized in that** during the manufacturing process with a magnesium master alloy, sintering is carried out at a temperature lower than the melting temperature of pure magnesium, whereby the proportional share of the liquid phase that is formed preferably should be kept at less than 20 %.

12. Process according to Claim 10 or 11, **characterized in that** a magnesium oxide layer is formed at least in certain areas at the surface of the fibers with the help of anodic oxidation, plasma chemical oxidation or thermal oxidation.

13. Process according to one of the Claims 10 through 12, **characterized in that** the implant is brought into a final shape with the help of a separation process or a cutting process, especially a laser cutting process

## Revendications

1. Implant qui est constitué de magnésium ou d'un alliage à base de magnésium, **caractérisé en ce qu'**il est constitué de fibres de magnésium ou d'un alliage à base de magnésium, qui sont reliées les unes aux autres par des ponts frittés localement éloignés les uns des autres, et qui forment un corps à pores ouverts.

2. Implant selon la revendication 1, **caractérisé en ce que** les fibres présentent une longueur comprise dans la plage de 2 mm à 15 mm, de préférence dans la plage de 5 mm à 10 mm, et/ou un diamètre extérieur compris dans la plage de 0,05 mm à 0,5 mm.

3. Implant selon la revendication 1 ou 2, **caractérisé en ce que** l'alliage à base de magnésium est formé avec au moins un métal avec lequel une phase liquide peut être formée à une température inférieure à la température de fusion du magnésium pur.

4. Implant selon l'une des revendications précédentes, **caractérisé en ce que** l'alliage à base de magnésium contient au moins un métal qui est choisi parmi Y, Zn, Ca, Mn, Pd, Ag, Sr, Bi, Si, Pr, Ga, Sc, Zr, Ce, Eu, La, Nd, Na et Li.

5. Implant selon l'une des revendications précédentes, **caractérisé en ce qu'**il présente une porosité comprise dans la plage de 50 % à 95 % et/ou une aire volumique spécifique comprise dans la plage de 5000 m²/m³ à 50 000 m²/m³.

6. Implant selon l'une des revendications précédentes, **caractérisé en ce que**, sur la surface des fibres, une couche d'oxyde de magnésium est formée au moins par zones, qui présentent de préférence une épaisseur de couche d'au moins 0,1 µm.

7. Implant selon l'une des revendications précédentes, **caractérisé en ce que**, sur la surface des fibres, une couche de fluorure est formée au moins par zones, qui de préférence présente une épaisseur de couche d'au moins 0,1 µm.

8. Implant selon l'une des revendications précédentes, **caractérisé en ce que** sont présentes des zones ayant une porosité et une résistance mécanique différentes.

9. Implant selon l'une des revendications précédentes, **caractérisé en ce qu'**il est relié à un implant supplémentaire.

10. Procédé de fabrication d'un implant en magnésium ou en un alliage à base de magnésium, dans lequel
des fibres sont fabriquées dans une atmosphère inerte par un procédé d'extraction à l'état fondu à partir d'une masse fondue de magnésium ou d'une masse fondue d'un alliage à base de magnésium ;
les fibres sont placées dans une masse en vrac, sur un support ou dans un moule, qui est constitué d'un matériau inerte, puis, dans une atmosphère inerte, on procède à un frittage, à l'occasion de laquelle les fibres sont reliées les unes aux autres par des ponts frittés localement éloignés les uns des autres.

11. Procédé selon la revendication 10, **caractérisé en ce que**, lors de la fabrication avec un alliage à base de magnésium, le frittage est mis en oeuvre en-dessous de la température de fusion du magnésium pur, ce à l'occasion de quoi la proportion de la phase liquide formée doit de préférence être maintenue inférieure à 20 %.

12. Procédé selon la revendication 10 ou 11, **caractérisé en ce que**, à l'aide d'une oxydation anodique, d'une oxydation chimique par plasma ou d'une oxydation thermique, il se forme sur la surface des fibres, au moins par zones, une couche d'oxyde de magnésium.

13. Procédé selon l'une des revendications 10 à 12, **caractérisé en ce que** l'implant est mis sous sa forme finale par un procédé de séparation ou de coupe, en particulier par coupe laser.
